# EUROPEAN PATENT SPECIFICATION

(11) **EP 0 473 128 B1**
(45) Date of publication and mention of the grant of the patent: **17.04.1996**
(21) Application number: 91114433.5
(22) Date of filing: 28.08.1991
(51) Int. Cl.: C12N 5/20, C12N 15/62, C12P 21/08, A61K 39/395

(54) **Monoclonal antibody specifically reactive with human perforin**
Monoklonaler Antikörper, der spezifisch mit dem humanen Perforin reagiert
Anticorps monoclonal réagissant spécifiquement avec la perforine humaine

(30) Priority: 28.08.1990 JP 227504/90
(43) Date of publication of application: 04.03.1992
(73) Proprietor: SUMITOMO ELECTRIC INDUSTRIES, LTD., Osaka-shi, Osaka 541 (JP)
(72) Inventor: Nakata, Motomi c/o Osaka Works, Konohana-ku Osaka-shi Osaka (JP); Maeda, Keiko, 1-1, Hongo 2-chome, Bunkyo-ku, Tokyo (JP); Okumura, Ko, 1-1, Hongo 2-chome, Bunkyo-ku, Tokyo (JP)
(74) Representative: Kolb, Helga, Dr. Dipl.-Chem.

(56) References cited:
- TISSUE ANTIGENS vol. 35, no. 5, May 1990, Copenhagen (DK); M. GEISBERG et al., pp. 229-233
- COMPLEMENT & INFLAMMATION, vol. 6, no. 5, 1989, Basel (CH); M. BROTHERS et al., p. 318

## Description

The present invention relates to a process for preparing a hybridoma capable of producing a monoclonal antibody specifically reactive with human perforin.

Perforin is a protein contained in cytoplasmic granules existing in Killer lymphocytes (hereinafter referred to as K cell), and the protein is released from K cell when the cell attackes and destroys its target cell. The released perforin binds to cell membrane of the target cell and polymerizes like "staves" in the presence of calcium ion, thereby it makes a pore in the cell membrane and let the target cell die due to the penetration of water and salts into the cell. Accordingly, enhancement of the pore-forming ability of perforin would provide an efficient means to treat carcinoma, AIDS, and other diseases.

On the other hand, it is known that amebic dysentery, some parasites, fungi, and bacteria produce a perforin-like protein which makes a pore in cell membrane like perforin. Accordingly, finding a means to inhibit the bio-synthesis of the perforin-like protein or the pore-making action of the protein would provide an efficient therapeutics for treating various diseases caused by the protein.

In such a situation, extraction of perforin from various K cells and its purification have been tried so that extensive studies on perforin can be done (see Young et al, Cell, 44, pp849-859, 1986; Okumura et al, Eur. J. Immunol., 18, pp29-33, 1988). In addition, there has been an attempt to obtain an anti-perforin antibody using a rabit antiserum against mouse perforin for the purpose of treating diseases associated with perforin or perforin-like substances and also for the purpose of accumulating knowledge about biological and immunological significance of perforin (see Young et al, Exp. Med., 169, pp2159-71, 1989). However, purified human perforin had not been isolated yet because of the following difficulties; (1) purification of human perforin requires highly trained technique because it exists only in granules of K cells; (2) purification procedure comprises very complicated processes; and (3) human perforin is chemically instable.

On the other hand, a gene encoding human perforin has been isolated with the help of mouse perforin gene disclosed in Japanese Patent Publication No. 2350/1989, and the base sequence of the gene and the amino acid sequence deduced therefrom have been known (see Shinkai et al, Immunogenetics, 30, pp452-457, 1989). The above-noted Japanese Patent Publication No. 2350/1989 discloses the mouse perforin gene isolated from K cells, an expression vector containing, integrated therein, the perforin gene, a host cell transformed with the expression vector, and a process for the preparation of a recombinant mouse perforin by culturing the transformed host cell. In addition, Podack et al succeeded in obtaining a perforin gene from another K cells (see Nature, 335, pp444-451, 1988).

Geisberg et al discloses monoclonal antibodies detecting discrete epitopes of human perforin (see Tissue Antigens, vol. 35, no. 5, pp. 229-233, May 1990).

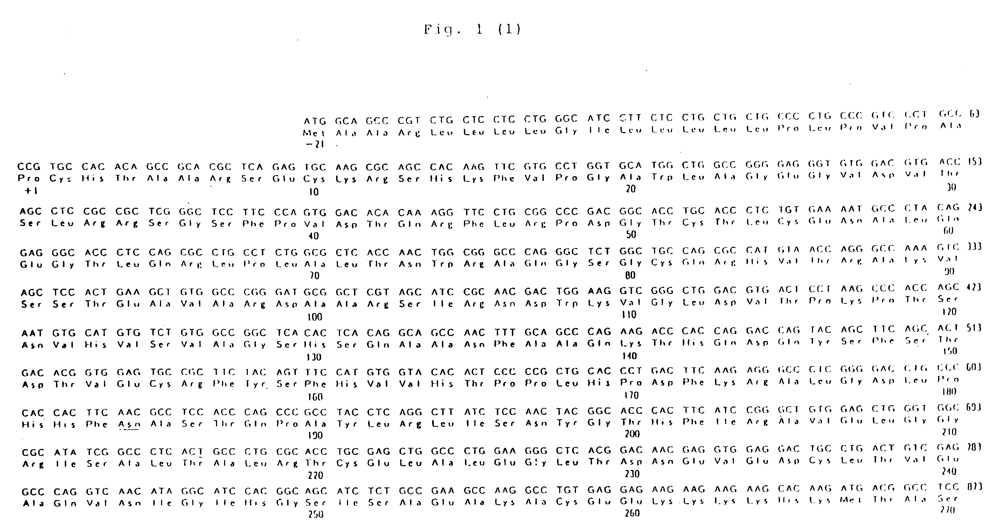

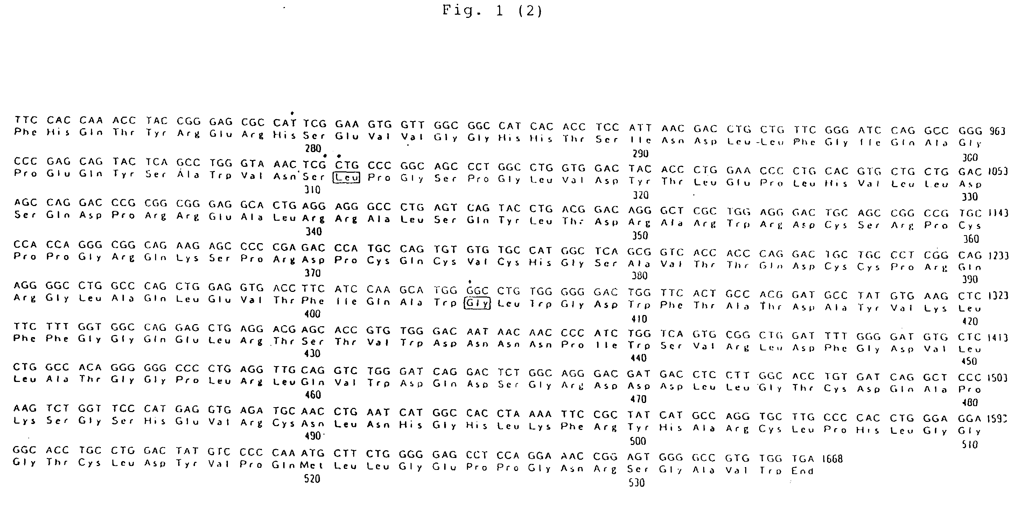

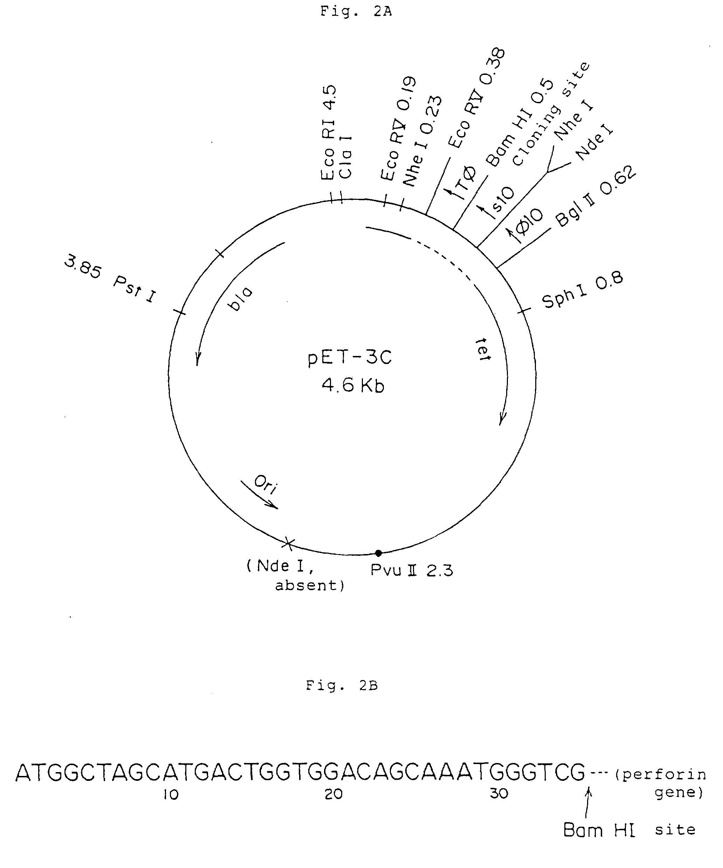

The present inventors have made extensive study in an attempt of obtaining anti-human perforin antibody, which study consisted of (1) integrating the 35 base sequence depicted in Fig. 2B of the accompanying drawings at N-terminal of the base sequence encoding an amino acid sequence of from 297 position (inclusive) to 463 position (inclusive) of human perforin depicted in Fig. 1 of the accompanying drawings, said partial amino acid sequence being expected to be unique and specific to human perforin, (2) introducing the combined base sequences encoding fused partial human perforin into an expression vector, (3) allowing the vector to express in E. coli cells, (4) purifying the expressed fused partial perforin, (5) immunizing a mouse with the perforin, and (6) obtaining desired mouse cells which produce an antibody reactive to the fused partial perforin. The inventors have found that a hybridoma prepared in conventional manner by the use of the mouse cells obtained above successfully produces anti-human perforin monoclonal antibody specifically reactive with human perforin. The present invention is based on such finding.

Thus, the present invention is to provide a process for preparing a hybridoma producing a monoclonal antibody specifically reacting with human perforin, which hybridoma is capable of being prepared by a method comprising the following steps: integrating a base sequence encoding an amino acid sequence of from 297 position (inclusive) to 463 position (inclusive) of human perforin depicted- in Fig. 1 of the accompanying drawings into an expression vector pET3C depicted in Fig. 2A which contains 35 base sequence depicted in Fig. 2B; transforming E. coli cells with the resultant expression vector; allowing the resultant transformant to produce a fused partial human perforin; immunosensitizing a rodentine animal with the fused partial human perforin; and fusing a spleen cell or lymphonodus cell obtained from the sensitized rodentine animal with a mouse myeloma cell to prepare the hybridoma.

The monoclonal antibody produced by the above-mentioned hybridoma, is characterized in that the antibody specifically reacts with human perforin, that the antibody belongs to IgG_{2b}, and that the L chain of the antibody is of κ type.

In the accompanying drawings:

Fig. 1 shows a base sequence encoding human perforin and an amino acid sequence deduced therefrom.

Fig. 2A is a restriction map of expression vector pET3C.

Fig. 2B shows 35 base sequence existing at 3' terminal of the cloning site indicated in Fig. 2A.

Fig. 3 shows electrophoresis of the monoclonal antibody of the invention.

The hybridoma and the monoclonal antibody of the invention may be prepared tan the following manner.
(i) Partial perforin gene, which is presumed to encode major perforin activity and also presumed to be unique to human perforin, is excised from human perforin gene, said partial perforin gene is integrated into an expression vector, the expression vector is incorporated into E. coli cells, and the transformed cells are allowed to produce the partial protein of human perforin.
(ii) The partial protein produced in Step (i) is extracted and purified, and a rodentine animal is immunosensitized with the purified partial protein.
(iii) A lymphonodus cell from the immunosensitized animal is fused with a mouse myeloma cell in conventional manner, and hybridomas producing a monoclonal antibody reactive with the human partial perforin is selected.
(iv) The hybridomas obtained in Step (iii) are cultured under appropriate conditions and monoclonal antibodies are recovered. Hybridomas expressing human perforin gene are subjected to immunohistostaining with the use of the obtained monoclonal antibody so that hybridomas producing a monoclonal antibody reactive with human perforin may be finally selected.
(v) The hybridomas obtained in Step (iv) are cultured to obtain the desired monoclonal antibody.

The monoclonal antibody thus obtained is capable of specifically reacting with human perforin molecule, and therefore, find a wide variety of applications. First, it is possible to identify K cells because the perforin molecule is expressed exclusively by K cells. Such identification yields various practical usage. Thus, it become possible to know the role of K cells by immunohistostaining of the diseased part in various diseases, which will reveal the major cause of the diseases which has not been known so far.

Rejection in transplantation occurs due to destruction of the transplanted tissue by Killer T cells (KT cells). If perforin immunohistostaining becomes possible by the use of the monoclonal antibody of the present invention, important information will probably be obtained which is very helpful in analyzing the rejection mechanism.

If it is found that perforin is involved in major reaction in the rejection, it would be possible to control the rejection by administation of the monoclonal antibody of the invention in transplant fashion, and to succeed in the transplantation with higher possibility.

The following detailed examples are presented by way of illustration of certain specific embodiments of the invention.

### Example 1

(1) Preparation of an expression vector into which a gene encoding partial human perforin has been integrated and preparation-of E. coli transformant BL21 (DC-3, lys S)

In the human perforin gene depicted in Fig. 1, a cDNA encoding the amino acid sequence of from 297 position to 463 position was excised and isolated using restriction enzyme.

The restriction enzyme Sau 3A recognizes the base sequence of GATC and digests the sequence in the following manner 
- CTAG -
- GATC -
 The use of this restriction enzyme enabled to excise a base sequence begining from one base before the codon encoding the amino acid of 297 position and ending at one base after the codon encoding the amino acid of 463 position. Specifically, Bluescript vector (available from Stratagene) (1µg/µl) containing integrated therein a human perforin gene (1µ) was combined with restriction enzyme Sau 3A (10U/ml) (1µl) and restriction H buffer (available from Takara Shuzo) (1µl), and the mixture was allowed to react at 37°C overnight to effect the excision.

The resultant mixture was subjected to 1% LMP (Low Melting Point) agarose gel (available form BRL) electrophoresis for 30 minutes using TAE buffer (Tirs-Acetate-EDTA buffer comprising 0.04M Tris-acetate and 0.001M EDTA). After DNA staining by the use of ethidium bromide, a gel fraction containing human perforin gene consisting of about 500bp was excised by a cutter under a UV monitor. The separated gel was suspended in 2.5 times by weight of TAE, and the gel was dissolved at 65°C. The TAE solution was extracted with phenol saturated with water which was warmed to 65°C. The phenol extraction was conducted three times, followed by chloroform extraction two times. To the residue were added 1/10 part by weight of 3M NaOAc buffer, 2.5 part by weight of ethanol, and 1µl of Mg (20 mg/ml). The mixture was allowed to stand at -80°C for 3 minutes to precipitate DNA. Centrifugation of the precipitated DNA at 15,000 rpm for 5 minutes was conducted for sedimentation of the DNA, which was then dissolved again in TE buffer (Tris-EDTA buffer comprising 10mM Tris-HCl pH8.0 and 1mM EDTA pH8.0) (20µl).

The Sau 3A-restricted perforin gene thus obtained was integrated into an expression vector pET3C (Rosenberg, BNL Laboratory). The vector pET3C is characterized in that it contains a starting coding region as shown in Fig. 2A and described in detail in Rosenberg et al, Gene, 56, pp125-135, 1987. The vector plasmid contains BamHI restriction site at the site corresponding to the 12th amino acid of the starting coding region. The base sequence of the remaining restriction end after excision by BamHI is GATC which is complementary to the remaining restriction end of partial human perforin fragment which has been formed by excision by Sau 3A. Thus, a mixture of pET3C (1µg), restriction enzyme BamHI (1µl), restriction K buffer (20mM Tris-HCl pH8.5, 10mM MgCl₂, 1mM Dithiothreitol, and 100mM KCl) (available from Takara Shuzo) (1µl), and distilled water (6µl) was allowed to react overnight at 37°C to effect linealization.

After addition of ethanol (30 µl), the reaction mixture was allowed to stand at -80°C for 2 hours, and then centrifuged at 15,000 rpm for 15 minutes to recover pET3C vector. The vector was dissolved in distilled water (2µl). To the solution were added alkaline phosphatase (Calf intestine, available from Takara Shuzo) (0.1µl) (10U/µl), Tris-HCl buffer (100mM Tris-HCl, pH 8.3, 10mM ZnCl₂, 10mM MgCl₂) (1µl) and distilled water (7µl), and the mixture was allowed to react at 37°C for 30 minutes. Proteinase K (available from Boehringer-Mannheim) (1µl) (10mg/ml) was then added thereto, and the mixture was allowed to react at 37°C for 30 minutes. The reaction mixture was extracted with phenol saturated with water, followed by ethanol precipitation to give a dephosphorylated single-stranded pET3C vector.

To the Sau 3A restriction fragment encoding partial human perforin (1µg/µl) (1µl) and linealized dephosphorylated single-stranded BamHI-restricted pET3C (1µg/µl) (1µl) were added 5 x ligase buffer (1µl), T4 DNA ligase (3.5U/ul) (1µl) and distilled water (5µl), and the mixture was allowed to react at room temperature for one hour so that Sau 3A-restricted partial perforin gene may be integrated into pET3C vector.

The thus obtained expression vector containing partial human perforin gene (PFP-pET3C) was used to transform E. coli BL21 (DC-3, lys S). For this purpose, a competent E. coli BL21 was employed. Preparation of competent cells was conducted in conventional manner (see Maniatis et al, Molecular Cloning, 2nd Edition). To competent BL21 (1 x 10⁶) was added PFP-pET3C (10µg) (100µl), and the mixture was allowed to stand under ice-cooling for 30 minutes. After 5 minutes incubation at 37°C, LB medium (1ml) containing tripsin (10g/l), yeast extract (5g/l), and NaCl (10g/l) was added, and then the mixture was incubated at 37°C for one hour. One minute centrifugation at 3000 rpm recovered BL21, to which fresh LB medium (100µl) was added. The mixture was plated on LB agar containing ampicillin, and grown BL21 cells were selected. The BL21 transformants were cultured on LB medium (2ml), and plasmid DNA was prepared from the culture by means of the alkali method (see Maniatis et al, Molecular Cloning). After BamHI restriction treatment, the lysate was subjected to agarose gel electrophoresis, and BL21 transformant containing partial human perforin gene was selected.

### (2) Production of partial human perforin

E. coli BL21 transformant which contains the expression vector bearing partial huamn perforin gene was plated on LB meidum (2.5ml) containing 150µg/ml of ampicillin and the medium was cultured at 37°C overnight. The transformant was then subcultured on LB medium (250ml) containing 150µg/ml of ampicillin, and the culture was continued for 2-2.5 hours until OD₆₀₀ becomes 0.6. After addition of IPTG (isopropylthio-β-D-galactoside) to a final concentration of 0.4mM, the culture was continued for 3 hours. Centrifugation at 6000 rpm for 5 minutes recovered the E. coli cells. The supernatant was discarded. To the cell pellet was added 5ml of Buffer A (50mM Tris-HCl, pH 8, 2mM EDTA, 1mM dithiothreitol, 1mM PMSF (Phenylmethylsulfonyl Fluoride), 5% glycerol) and the mixture was mixed well.

To the mixture was added lyzozyme (available from Sigma) to a final concentration of 0.2mg/ml and the mixture was allowed to gently stand at 0°C for 30 minutes, and the cells were destructed by means of ultrasonic treatment (Sonfier Cell Disruptor 200, Branson) (15 minutes x 4).

Triton X-100 was added to a final concentration of 0.5% and the mixture was allowed to gently stand at 0°C for 20 minutes. The mixture was then centrifuged at 10,000 x g for 45 minutes at temperature of 4°C to obtain a crude extraction of fused partial human perforin.

The crude extract containing the fused partial perforin was subjected to SDS-polyacrylamide gel electrophoresis and the fused partial perforin of 19.8 K dalton was recovered.

### (3) Preparation of monoclonal antibody

### A. Immunosensitization

The fused partial human perforin (50µg) obtained above was mixed with complete Freund's adjuvant at a ratio of 1:1 to make an emulsion, which was then injected to Balb/c mice (Charles River) at their foot pad. Six and nine days after the injection, the fused partial human perforin (50µg) was injected in the foot pad of the same animals for immunosensitization.

### B. Hybridization

Three days after the final injection, lymphonodus was removed from the inguen of both legs of two mice, and the lymphonodus was cut into small pieces, filtered on a mesh, and allowed to float on RPMI 1640 medium to obtain 8 x 10⁷ lymphonodus cells. A mouse-derived 8-azaguanidine-resistant strain PAI (1.3 x 10⁷) (hypoxanthine guanine phophoribosyl transferase-deficient strain) was mixed with the above-obtained lymphonodus cells at a ratio of 1:6, and the mixture was centrifuged (1500 rpm, 5 minutes). To the resultant precipitates, a 50% polyethylene glycol 4000 (available from Merk)/RPMI 1640 solution (3ml) was added at 37°C over one minute with stirring on a warm water bath. RPMI 1640 (15ml) was added with stirring over six minutes to allow the hybridization. After completion of the hybridization, a lot of RPMI 1640 was added, and centrifugation (1500 rpm, 5 minutes) was conducted and the supernatant was discarded.

The cell pellet thus obtained was cultured in a 15%-Nu-serum-RPMI 1640 medium (HAT medium, Collaborative Research Incorporated) containing hypoxanthine (100µM), aminopterin (0.4µM) and thymidine (10µM) so that the lymphonodus cells may amount to 1 x 10⁶/ml.

### C. Selection of hybridoma

The cells-floating medium prepared above was placed on five 96-well microplates (200µl each) and cultured at 37°C in a CO₂-incubator which was kept at below 5% CO₂ gas level. After six days, the formation of colony of hybridoma was confirmed.

### D. Detection of antibody

Since it has been confirmed that sufficient amount of hybridoma cells are grown, enzyme-linked immunosorbent assay (ELISA) was conducted on the culture medium to detect the antibody. Thus, 50µl of a lysate (crude extract) (5.32mg/ml) of E. coli cells which expressed and produced fused partial human perforin and 50µl of a lysate (crude extract) (2.1mg/ml) of E. coli cells which did not expressed and produced the same were separately charged on an assay plate (Dinatech. Immuron 2) and allowed to stand overnight at 4°C so that possible antigen may be fixed on the plates. The lysates were discarded, and a blocking reagent (Block Ace, Dainippon Seiyaku K.K.) was added to the plates. The culture medium of the hybridoma (50µl) was added to each plate and allowed to react for one hour at room temperature.

The plates were then washed with PBS containing 0.05% Tween 20, and to the plates was added 50µl of one thousandth (1/1000) dilution of F(ab)₂' fragment of anti-mouse Ig antibody (Amersham) which was labeled with peroxidase (horse radish, HRP) so that antigen-antibody reaction may occur for one hour at room temperature. The plates were washed again with PBS, and to the plates was added a phosphate-citrate buffer (pH 5.0) (100µl) containing o-phenylenediamine (0.4mg/ml) and hydrogen peroxide (0.012%) so that the antibody may be detected through color development. Based on the test, a well, which produced an antibody reactive to E. coli lysate containing fused partial human perforin and non-reactive to E. coli lysate containing no fused partial human perforin, was selected.

### E. Cloning

Hybridomas in the well selected in the above step D were cloned by means of limiting dilution analysis. Thus, the well was diluted with 15% Nu-serum-RPMI 1640 medium so that the final cell concentration may be 0.5 cell/well, and the dilution was portioned on a 96-well plate at a ratio of 200µl/well. The plate was incubated at 37°C in the presence of 5% CO₂. When colonies have grown, the enzyme immunoassay described in the above step D was conducted again in order to examine the production of the antibody against the partial human perforin. A hybridoma in the well which showed strong reaction with the partial human perforin was cloned again by the use of the limiting dilution analysis.

### F. Reactivity with human perforin

Immunohistological stain was conducted in the following manner to determine if the antibody produced by the hybridoma obtained above reacts with human perforin.
(1) Human LAK (lymphokine activated killer) cells activated with human peripheral blood lymphocyte and IL-2 were fixed on a slide glass using Cytospin 2 (Shandon). The human peripheral blood lymphocyte was isolated from peripheral blood of normal volunteer by means of separate L (Muto Kagaku) specific gravity centrifugal separation according to the method disclosed in Boyum. Scand. J. Clin. Lab., Ln rest, 21 (Suppl. 97): 77, 1968. The activation of LAK cells was conducted by culturing the cells for 4 days in 10% FCS-RPMI 1640 medium containing the human peripheral blood lymphocyte obtained above and IL-2 (Shionogi) (1000U/ml).
(2) The slide glass in step (1) was dipped in acetone for fixation at -20°C for 3 minutes and then dipped in a 4% paraformaldehyde/PBS solution for one minute to complete the fixation.
(3) The slide glass was dipped in 50mM Tris-HCl, pH 7.6, for 5 minutes and then washed.
(4) The slide glass was then dipped in a 0.5% periodic acid solution in distilled water for 10 minutes to effect denaturation.
(5) The slide glass was washed again two times with 50mM Tris-HCl, pH 7.6, for 5 minutes.
(6) The slide glass was dipped in methanol containing 0.3% hydrogen peroxide in order to inactivate peroxidase existing in the cells.
(7) The slide glass was then washed two times with Tris-HCl.
(8) Rabbit serum was diluted 50 times by 1% BSA (bovine serum albumin)-PBS, and the dilution was poured on the fixed cells (each 50µl) for blocking (30 minutes, room temperature).
(9) The culture medium of hybridoma obtained in the previously-mentioned Step E (100µl) was added thereto and allowed to react at room temperature for one hour.
(10) The slide glass was washed three times with Tris-HCl.
(11) Anti-mouse IgG (H+L) labeled with biotin (Vector) was diluted 100 times, added to the cells (each 50µl), and allowed to react at room temperature for one hour.
(12) The slide glass was treated with Tris-HCl three times.
(13) Binding of peroxidase was conducted by the use of Vectastain's ABC Kit Elite (Vector) (30 minutes, room temperature).
(14) The slide glass was treated with Tris-HCl three times.
(15) The slide glass was dipped in 50mM Tris-HCl (100ml), pH 7.6, containing diaminobenzidine (20mg) and 30% hydrogen peroxide (10µl) for color development.

As the results of the above procedures, there were selected two hybridomas which produce monoclonal antibody capable of selectively staining human perforin existing in granules in human peripheral blood lymphocytes and some LAK cells. These two hybridomas, designated as MPFP-1 and MPFP-2, were considered identical species. One of them, MPFP-1, was deposited on August 20, 1990, at Fermentation Research Institute Agency of Industrial Science and Technology, 1-3, Higashi 1 chome, Tsukuba-shi, Ibaraki-ken, Japan, under Accession Number of FERM P-11669, and then the deposition was converted to the deposition under Budapest Treaty on August 14, 1991 and assigned Accession No. Bikokenjoki 3514 (FERM BP-3514).

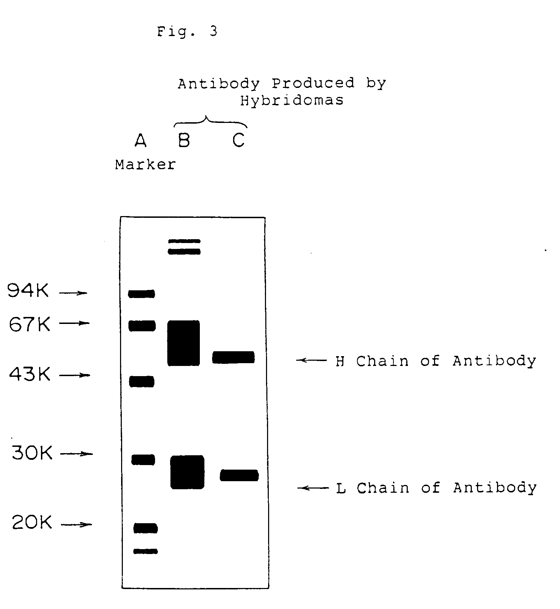

Each of the hybridomas thus obtained (1 x 10⁷ cells) was cultured in GIT medium (Wakojunyaku) (100ml) for 3 days. The culture medium was centrifuged at 1500 rpm for 5 minutes to recover the supernatant, which was then charged in Protein G column (Mab trap G, Pharmacia) to obtain the aimed antibody. The antibody was subjected to 10% SDS-polyacrylamide gel electrophoresis. Protein-staining was conducted using Coomassie Brilliant Blue to detect the antibody, which revealed that both of the antibodies derived from the two hybridomas are IgG consisting of H chain having a molecular weight of 50,000 and L chain of 25,000. Fig. 3 of the accompanying drawings shows SDS-polyacrylamide gel electrophoresis, in which Lane A shows molecular weight marker (90K, 67K, 43K, 30K, 20K), Lanes B and C show the antibodies from hybridomas MPFP-1 and MPFP-2, respectively.

### G. Determination of subclasses of the antibodies

Subclass of each of the antibodies produced by two hybridomas was determined by enzyme immunoassay wherein the anti-human perforin monoclonal antibody in hybridoma culture medium is allowed to react with anti-mouse antibodies (anti-IgG₁, anti-IgG₂ₐ, anti-IgG_{2b}, anti-IgG₃, anti-IgM, and anti-IgA antibodies) and anti-mouse L chain antibodies (anti-κ and anti-λ antibodies) (MAB-Isotypic Kit, Pharmingen). Color development was observed only on anti-IgG_{2b} antibody and anti-κ antibody with respect to both antibodies, which revealed that the two hybridomas produce a monoclonal antibody belonging to subclass IgG_{2b} and having L chain of κ type.

## Claims

1. A process for preparing a hybridoma capable of producing a monoclonal antibody specifically reacting with human perforin, comprising the following steps:
(i) integrating a base sequence encoding an amino acid sequence of from 297 position (inclusive) to 463 position (inclusive) of human perforin into an expression vector pET3C according to Fig. 2A at BamHI restriction site, said expression vector carrying the following 35 base sequence which partially constitutes said BamHI restriction site; 
ATGGCTAGCATGACTGGTGGACAGCAA
10 20
ATGGGTCG
30 ↑
. BamHI site
(ii) transforming E. coli cells with the thus modified expression vector,
(iii) allowing the resultant transformant to produce a fused partial human perforin,
(iv) immunosensitizing a rodentine animal with the fused partial human perforin, and
(v) fusing a spleen cell or lymphonodus cell obtained from the sensitized rodentine animal with a mouse myeloma cell to prepare the hybridoma.

2. The process according to claim 1, wherein the mouse myeloma cell is a PAI myeloma cell and the lymphonodus cell is derived from a BALB/c mouse.

3. A process for preparing a monoclonal antibody comprising culturing the hybridoma of claim 1 and collecting the produced monoclonal antibody, characterized in that the antibody specifically reacts with human perforin, that the antibody belongs to IgG_{2b}, and that the L chain of the antibody is of kappa type.

## Patentansprüche

1. Verfahren zur Herstellung eines Hybridoms, das in der Lage ist, einen monoklonalen Antikörper zu produzieren, der spezifisch mit Humanperforin reagiert, wobei das Verfahren die folgenden Schritte umfaßt:
(i) Integrieren einer Basensequenz, die eine Aminosäuresequenz von Position 297 (einschließlich) bis Position 463 (einschließlich) von Humanperforin codiert, in einen Expressionsvektor pET3C entsprechend Fig. 2A bei der BamHI Restriktionsstelle, wobei dieser Expressionsvektor die nachfolgend dargestellte 35-Basen-Sequenz aufweist, die teilweise die erwähnte BamHI-Restriktionsstelle bildet; 
ATGGCTAGCATGACTGGTGGACAGCAA
10 20
ATGGGTCG
30 ↑
BamHI-Stelle
(ii) Transformieren von E. coli-Zellen mit dem so erhaltenen modifizierten Expressionsvektor;
(iii)man läßt die erhaltenen Transformante ein verschmolzenes partielles Humanperforin produzieren;
(iv) Immunosensibilisieren eines Nagetiers mit dem verschmolzenen partiellen Humanperforin; und
(v) Verschmelzen einer von dem sensibilisierten Nagetier erhaltenen Milzzelle oder Lymphknotenzelle mit einer Mäusemyelomzelle zurHerstellung des Hybridoms.

2. Verfahren nach Anspruch 1, wobei die Mäusemyelomzelle eine PAI-Myelomzelle und die Lymphknotenzelle von einer BALB/c-Maus abgeleitet ist.

3. Verfahren zur Herstellung eines monoklonalen Antikörpers, wobei dieses Verfahren die Schritte des Kultivierens des Hybridoms nach Anspruch 1 und Sammeln der produzierten monoklonalen Antikörper umfaßt, dadurch gekennzeichnet, daß der Antikörper spezifisch mit Humanperforin reagiert, der Antikörper der Klasse IgG2b angehört und die L-Kette des Antikörpers vom Typ κ ist.

## Revendications

1. Procédé de préparation d'un hybridome capable de produire un anticorps monoclonal réagissant spécifiquement avec une perforine humaine, comprenant les étapes suivantes:
(i) intégrer une séquence de bases codant une séquence d'acides aminés de la position 297 (incluse) à la position 463 (incluse) de la perforine humaine, dans un vecteur d'expression pET3C selon la figure 2a, sur un site de restriction BamHI, ledit vecteur d'expression transportant la séquence de 35 bases ci-dessous, qui constitue partiellement ledit site de restriction BamHI: 
ATGGCTAGCATGACTGGTGGACAGCAA
10 20
ATGGGTCG
30 ↑
site BamHI
(ii) transformer des cellules de E. coli avec le vecteur d'expression ainsi modifié,
(iii) laisser le transformant résultant produire une perforine humaine partielle fusionnée,
(iv) immunosensibiliser un rongeur avec la perforine humaine partielle fusionnée, et
(v) fusionner une cellule de rate ou une cellule de noeud lymphatique tirée du rongeur sensibilisé, avec une cellule de myélome de souris, pour préparer l'hybridome.

2. Procédé selon la revendication 1, dans lequel la cellule de myélome de souris est une cellule de myélome PAI, et la cellule de noeud lymphatique est tirée d'une souris BALB/c.

3. Procédé de préparation d'un anticorps monoclonal comprenant une mise en culture de l'hybridome selon la revendication 1 et la récolte de l'anticorps monoclonal produit, caractérisé en ce que l'anticorps réagit spécifiquement avec la perforine humaine, en ce que l'anticorps appartient à IgG_{2b}, et en ce que la chaîne L de l'anticorps est du type kappa.
